(19) Europäisches Patentamt
European Patent Office
Office européen des brevets

(11) EP 1 978 361 A2

(12) EUROPEAN PATENT APPLICATION

(43) Date of publication:
**08.10.2008 Bulletin 2008/41**

(51) Int Cl.:
***G01N 33/50*** *(2006.01)*

(21) Application number: **08006729.1**

(22) Date of filing: **02.04.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **06.04.2007 US 922089 P**

(71) Applicant: **Bayer HealthCare, LLC**
**Tarrytown, NY 10591 (US)**

(72) Inventors:
- **Chu, Amy H.**
  **Elkhart, IN 46514 (US)**
- **Warchal-Windham, Mary Ellen**
  **Osceola, IN 46561 (US)**

(74) Representative: **Linhart, Angela**
**Bayer HealthCare AG**
**Law and Patents, Patents and Licensing**
**51368 Leverkusen (DE)**

# (54) Method for performing correction of blood glucose assay bias using blood hemoglobin concentration as determined by a glucose-hemoglobin A1C combination system and devices for same

(57) A device and method for determining a correction factor for correcting the blood glucose assay bias based on sample hematocrit interference in a testing device using the blood hemoglobin concentration. The hemoglobin assay and the glucose assay may be performed using a single combination monitoring device.

EP 1 978 361 A2

## Description

## FIELD OF THE INVENTION

The invention relates to methods and devices for correcting the blood glucose assay bias in glucose-monitoring devices to provide more accurate glucose readings of blood samples. In particular, the invention relates to methods for using hemoglobin concentrations determined from a hemoglobin $A_{1C}$ assay to correct the glucose assay bias resulting from sample hematocrit interference. The hemoglobin $A_{1C}$ assay and the glucose assay may be performed using a single monitoring system. The system may include a meter and one or more testing devices, such as one or more test strips, one or more test cartridges, a combination of a test strip and test cartridge, etc.

## BACKGROUND OF THE INVENTION

Blood hematocrit is the ratio of the volume of packed red blood cells to the total blood volume, expressed as a percentage of the volume of red blood cells in a whole blood sample. The normal hematocrit range for a typical human being is about 35 vol.% to about 45 vol.%, though in extreme cases, the hematocrit may range from about 20 vol.% to about 70 vol. %. The mean hematocrit range for neonatal is about 53 vol.% to about 69 vol.%. Blood hematocrit levels outside of the normal range of 35 vol.% to 45 vol.% may interfere with blood glucose-monitoring systems which are based on either optical or electrochemical reaction principles. Hematocrit interference contributes to a glucose assay bias in glucose-monitoring systems, which can be as large as +/- 50% of the measured glucose concentration.

The glucose bias caused by the hematocrit content of a blood sample can have an adverse effect on patients. Patients who have low hematocrit levels (i.e., blood with about 35 vol.% hematocrit or lower) may misinterpret their glucose value as being too high because of the glucose bias and think they need insulin to bring their high glucose level down. Because the actual glucose level is not as high as the perceived glucose level, patients may drop their glucose level too low by unnecessarily taking too much insulin. Conversely, patients who have high hematocrit levels (i.e., blood with about 45 vol.% hematocrit or higher) may misinterpret their glucose value as being normal or low when it is actually high because of the glucose bias. Because the actual glucose level is not as low as the perceived glucose level, patients may consistently forego or miss needed treatment, leading to long term medical complications.

To minimize the hematocrit interference with the glucose assay, the blood sample may either be filtered to eliminate the red blood cells or the amount of red blood cells in the blood sample must be accurately determined. Although hematocrit values of whole blood samples have been measured routinely in the clinical or laboratory setting, hematocrit values are not commonly measured with hand-held, home-use, blood glucose-monitoring devices.

Some blood glucose-monitoring devices have been developed to determine hematocrit values of a whole blood sample in a glucose-monitoring device using a blood reflectance system for measuring the reflectance of the blood sample at a specific wavelength. The results are then used to normalize the glucose-concentration readings. Other monitoring devices use a transmission system wherein a blood sample is illuminated with light and the total transmission through the blood sample is collected and used to correct the glucose concentration based on the spectral properties of the hemoglobin of the blood sample. With the above devices, the spectral properties of the blood sample are measured through either light reflectance or transmittance using a specific wavelength where the optical property of hemoglobin of the red blood cells, which is less sensitive to other components in the blood, must be chosen to minimize the interference with other blood components. Typically, the amount of hemoglobin from the red blood cells is estimated optically at a wavelength of about 680 nanometers or other wavelengths characteristic of hemoglobin spectral properties. A correction for glucose can be made if the device also measures the spectral property of the hemoglobin molecule at about the 680 nanometer wavelength or other wavelengths characteristic of hemoglobin spectral properties.

Such systems for correcting glucose concentrations in a blood sample must have an optical reflectance or transmission system. However, some glucose monitoring devices are based on electrochemical reactions and do not employ optical systems. Thus, a desirable testing system would employ a method for adjusting glucose concentrations based on hematocrit interference that is not dependent on the type of glucose testing system employed. Furthermore, a device that is adapted to measure a combination of analytes (e.g., glucose and hemoglobin $A_{1C}$) is desirable as a patient has the convenience of monitoring current glucose concentrations to determine the long term glycemic control in a single device.

## SUMMARY OF THE INVENTION

According to one embodiment, a method for correcting the blood glucose assay bias in a testing device or system that measures glucose and hemoglobin concentrations in a blood sample comprises the acts of measuring the total hemoglobin concentration, Hb, of the blood sample as part of a hemoglobin $A_{1C}$ assay, measuring the glucose concentration, $Glu_{uncor}$, of the blood sample, and calculating the percent hematocrit, %Hct, of the blood sample based on an empirical relationship between the total hemoglobin concentration, Hb, and the percent hematocrit, %Hct. The method further comprises calculating a correction factor, %Correction, based on an empirical relationship between the percent hematocrit, %Hct and the correction factor, %Correction and correcting the glucose concentration, $Glu_{uncor}$, of the blood sample using the %Correction to provide a corrected glucose concentration, $Glu_{cor}$.

According to another embodiment, a testing device or system for measuring the blood glucose concentration and total hemoglobin concentration in a blood sample and correcting the measured glucose concentration for a glucose assay bias effect includes a reagent system adapted to measure the blood glucose concentration, $Glu_{uncor}$ of the blood sample and a reagent system adapted to measure the total hemoglobin concentration, Hb, of the blood sample as part of a hemoglobin $A_{1C}$ assay. The testing device or system also includes a processor adapted to calculate a percent hematocrit, %Hct, of the blood sample based on an empirical relationship using the total hemoglobin concentration, Hb, a correction factor, %Correction, based on an empirical relationship using the percent hematocrit, %Hct, and a corrected glucose concentration, $Glu_{cor}$, of the blood sample based on the correction factor, %Correction. The testing device or system further includes a display adapted to display the corrected glucose concentration, $Glu_{cor}$, to a user.

According to a further embodiment, a single combination testing device is adapted for measuring blood glucose concentration and total hemoglobin concentration in a blood sample, the total hemoglobin concentration being measured as part of a hemoglobin $A_{1C}$ assay.

The above summary of the present invention is not intended to represent each embodiment or every aspect of the present invention. The detailed description and Figures will describe many of the embodiments and aspects of the present invention.

## BRIEF DESCRIPTION OF THE DRAWINGS

The foregoing and other advantages of the invention will become apparent upon reading the following detailed description and upon reference to the drawings.

FIG. 1 is a top view of a test sensor according to one embodiment.

FIG. 2 is a top view of a test sensor according to another embodiment.

FIG. 3 is a schematic diagram describing a method for adjusting the blood glucose assay bias using blood hemoglobin concentration

FIG. 4 is a graph showing the relationship between blood hemoglobin concentration and % hematocrit level.

FIG. 5 is a graph showing the relationship between the % hematocrit level and the % glucose assay bias for samples at different glucose concentrations.

FIG. 6 is a graph showing the relationship between constant C3 and the "uncorrected" glucose concentrations.

FIG. 7 is a graph showing the relationship between constant C4 and the "uncorrected" blood glucose concentrations.

## DESCRIPTION OF EMBODIMENTS OF THE INVENTION

Embodiments of the invention are, in part, based on the discovery that the performance of glucose-monitoring devices or systems may be improved by adjusting the blood glucose value based on sample hematocrit level in glucose-monitoring devices or systems. It has been discovered that by using the methods and devices described herein, a more accurate glucose reading can be obtained. By obtaining a more accurate glucose reading, a more accurate assessment of the glycermic stage of a patient can be obtained and readily reported to the patient's physician.

As used herein, the term "blood glucose assay bias" is defined as a trend in the collection, analysis, interpretation, or review of glucose data from a glucose assay that leads to the conclusion that the patient's measured glucose level of the blood sample is systematically different from the patient's actual glucose level.

The present invention generally includes measuring the blood glucose concentration and the total blood hemoglobin concentration in a whole blood sample on the same testing device. The tests for measuring the blood glucose concentration and the total blood hemoglobin concentration may be performed using the same blood sample or a different blood sample. The total blood hemoglobin concentration of the red blood cells may be measured optically as part of a hemoglobin $A_{1C}$ assay. The total hemoglobin concentration can then be used to calculate the hematocrit level in the blood sample using an empirical relationship. The hematocrit level can in turn be used to calculate a correction factor using an empirical relationship. The correction factor is applied to the measured blood glucose concentration to calculate a corrected blood glucose concentration, which is reported or displayed to a patient. As the blood hematocrit levels are fairly constant from day to day, even for individuals with diabetes, the calculated hematocrit level can be stored in the testing device and used to correct the glucose test results. The calculated hematocrit level may be stored in the testing device until a new hematocrit level is determined which replaces the previous hematocrit level.

The measurement of the blood glucose concentration of a whole blood sample may be based on conventional methods using optical or electrochemical reaction principles. For example, a variety of sensors are known that use electrochemical principles to measure the amount of glucose in a blood sample, as shown in FIG. 1. FIG. 1 depicts a test sensor 70 that includes a capillary channel 72, an area for meter contacts 86, and a plurality of electrodes 76, 80, 84. The plurality of electrodes includes a counter electrode 76, a working (measuring) electrode 80, and an optional trigger electrode 84. The trigger electrode 84 may assist in determining whether a sufficient blood sample has been placed on the sensor 70. The electrochemical test sensor may also contain other numbers and/or types of electrodes.

The capillary channel 72 contains reagent. The reagent typically contains an enzyme such as, for example, glucose oxidase, which reacts with the analyte and with an electron acceptor such as a ferricyanide salt to produce an electrochemically measurable species that can be detected by the electrodes. It is contemplated that other enzymes may be used to react with glucose such as glucose dehydrogenase. Examples of electrochemical test sensors, including their operation, may be found in, for example, U.S. Patent No. 6,531,040 (entitled "Electrochemical-sensor Design") assigned to Bayer Corporation.

In other sensors, the optical properties of a whole blood sample are measured at a certain wavelenth(s) to determine the glucose concentration. FIG. 2 shows an optical test sensor 88 according to one embodiment. The test sensor 88 includes a base (not shown), a lid 89, and a spacer (not shown) positioned between the lid 89 and the base. When the base, the lid 89, and the spacer are attached together, a fluid-receiving area 90 is formed. The fluid-receiving area provides a flow path for introducing the fluid sample into the test sensor 88. The fluid-receiving area 90 is formed at a first end or testing end 91 of the test sensor 88. The test sensor 88 includes a second opposing end 92. The second opposing end 92 is adapted to be placed into a meter or instrument.

Optical test sensor systems, as shown in FIG. 2, may use techniques such as, for example, transmission spectroscopy, diffuse reflectance or fluorescence spectroscopy for measuring the analyte concentration. An indicator reagent system and glucose in a sample of body fluid are reacted to produce a chromatic reaction-the reaction between the reagent and glucose causes the reagent to change color. The degree of color change is indicative of the glucose concentration in the body fluid measured by the absorbance level of the transmitted light. Transmission spectroscopy is described in, for example, U.S. Patent No. 5,866,349 (entitled "Method for Determination of Glucose in Whole Blood and Cuvette and Photometer for Carrying out said Method"). Diffuse reflectance and fluorescence spectroscopy are described in, for example, U.S. Patents Nos. 5,518,689 (entitled "Diffuse Light Reflectance Read Head"); 5,611,999 (entitled "Diffuse Light Reflectance Read Head"); and 5,194,393 (entitled "Optical Biosensor and Method of Use"). It is contemplated that other optical and electrochemical test sensors may be employed.

According to one embodiment of the present invention, the measurement of the concentration of total hemoglobin occurs in the same testing device that is used for measuring the glucose concentration. The measurement of the total hemoglobin in the whole blood sample may be based on chemical reactions between the hemoglobin molecules and a chemical reagent. For example, one such method for measuring the total hemoglobin concentration includes the acts of: (1) lysing the red blood cells to release hemoglobin molecules, (2) complexing the hemoglobin molecules with the chemical reagents to form a colored species, and (3) measuring the extent of the color development at a particular wavelength.

One example of such a colorimetric method is the cyanmethemoglobin method. The principle of this method is based on the fact that when a sample of blood is mixed with a solution containing potassium ferricyanide and potassium cyanide, the potasium ferricyanide oxidizes iron of hemoglobin to form methemoglobin. The potassium cyanide then combines with methemoglobin to form cyanmethemoglobin, a stable color pigment which can be read photometrically at a wave length of about 540 nm. Some advantages of the cyanmethemoglobin method are that (1) all forms of hemoglobin, except sulfhemoglobin, are measured, (2) the method is easily standardized, (3) the cyanmethemoglobin reagent, also called Drabkin's solution, is very stable, and (4) the method may be used in combination with an immunological assay to determine the amount of hemoglobin $A_{1C}$ in a blood sample.

It is contemplated that other methods for measuring the concentration of hemoglobin in a whole blood sample are known in the art and may be used with the present invention, such as gasometric methods, specific gravity methods and chemical methods. With all of the methods mentioned herein, the hemoglobin and hemoglobin $A_{1C}$ measurement can be performed using the same testing device as the glucose measurement (a "combo" meter). A single drop of blood can be used for performing both the hemoglobin/hemoglobin $A_{1C}$ assay as well as the glucose assay.

Once the total hemoglobin concentration has been determined, this value may be used to calculate other relevant quantities. As shown in FIG. 3, the total hemoglobin concentration (Hb) can be used to calculate the hemoglobin $A_{1C}$ concentration. Hemoglobin $A_{1C}$ may be formed by an immunological reaction involving the non-enzymatic glycation of the N-terminus of the β-chain of hemoglobin. Other reactions to determine the hemoglobin $A_{1C}$ concentration may also be used with the present invention. The concentration of hemoglobin $A_{1C}$ is commonly reported as the percent hemoglobin $A_{1C}$, $\%HbA_{1C}$, in a blood sample. The percent hemoglobin $A_{1C}$, $\%HbA_{1C}$, is reported as the ratio of the concentration of hemoglobin $A_{1C}$ to the concentration of total hemoglobin. The percent hemoglobin $A_{1C}$, $\%HbA_{1C}$, is commonly used to monitor the long-term (i.e., 3-week) glycemic control for people with diabetes.

In addition to the hemoglobin $A_{1C}$ concentration, the total hemoglobin concentration is used to determine the hematocrit of the whole blood sample. The hematocrit may be used to correct the glucose assay bias as shown in FIG.

3. The hematocrit is the ratio of the volume of packed red blood cells to the total blood volume. Under normal conditions, there is a linear relationship between hematocrit levels and the concentration of total hemoglobin. The linear relationship may be described by an equation such as that shown in Equation 1:

$$\%Hct = C1 \times Hb + C2 \qquad \text{(Eq. 1)}$$

where Hb is the total hemoglobin concentration in gram/dL. C 1 and C2 are constants that may be determined from plotting hemoglobin concentration vs. % hematocrit using the hemoglobin assay systems described below and shown in FIG. 4. Based on the three assay systems described below and shown in FIG. 4, C1 equals about 2.95, which is the mean slope of the three systems shown in FIG. 4, and C2 equals about -2.0, which is the mean intercept of the three systems shown in FIG. 4.

This empirical relationship between the percent hematocrit, %Hct, and the total hemoglobin (Hb) concentration is based on studies performed using hemoglobin reagents (i.e., Drabkin's reagent) obtained from Sigma Chemical Co, HemoCue Ltd. and Bayer DCA 2000 analyzer. From those studies, the two constants, C1 and C2, were determined empirically based on the hemoglobin test results as plotted in FIG. 4. The values of the constants C1 and C2 are dependent on the specific reagent system that is used to determine the hemoglobin concentration. The 95% confidence limit for C1 ranges from about 2.2 to about 3.4 and for C2 ranges from about -9 to about 4, based on the evaluation of the three systems using the Drabkin's reagent discussed above. For the data shown in FIG. 4, the sample hematocrit levels were varied in the range of 0 to 60% and the average values of two replicates for each sample were used for plotting.

In embodiments of the present invention, the percent hematocrit, %Hct, determined from Equation 1 may be used to derive a correction factor, %Correction, for correcting the "apparent" glucose concentration, $Glu_{uncor}$., measured by the "combo" device before blood hematocrit correction. According to one embodiment, an empirical relationship between the percent hematocrit, %Hct, and the correction factor, %Correction, may be shown by

$$\%Correction = (\%Hct \times C3) + C4 \qquad \text{(Eq. 2)}$$

where %Hct is the % hematocrit determined from Equation 1 and C3 and C4 are constants that may be determined from plotting % hematocrit vs. % glucose assay bias based on the results of a blood glucose monitoring system using whole blood samples at different hematrocrit levels (i.e., 20%, 40% and 55%) as described below and shown in FIG. 5.

Based on the glucose assay systems shown in FIG. 5, the blood glucose concentrations for each hematocrit level was adjusted to 50, 100, 200 and 400 mg/dL. Twenty replicates per sample were collected. The mean assay bias at each glucose level was calculated by comparing glucose assay results obtained from samples with low (i.e., 20% hematocrit) and high (i.e., 55% hematocrit) hematocrit levels to the assay results of the 40% hematocrit sample. The data plotted in FIG. 5 is an average % assay bias from three lots of test sensors. C3 and C4 are equal to the mean slope and mean interecept, respectively, for each glucose assay performed and thus are dependent on the concentration of glucose in each sample. At each glucose concentration, the sample hematocrit levels were adjusted to 20%, 40% and 55%.

For example, for a sample containing 50 mg/dL glucose, C3 = about - 0.1478 and C4 = about 6.4338; for a sample containing 100 mg/dL glucose, C3 = about - 0.6041 and C4 = about 24.922; for a sample containing 200 mg/dL glucose, C3 = about - 1.010 and C4 = about 41.476; and for a sample containing 400 mg/dL glucose, C3 = about - 1.1591 and C4 = about 48.943. Using these slope and intercept values, the relationship between C3 and C4 and the respective glucose concentration can be further expressed in Equations 3 and 4 below which were derived based on the correlation plots shown in FIGS. 6 and 7:

$$C3 = -0.4962 * \ln(Glu_{uncor}) + 1.7269 \qquad \text{(Eq. 3)}$$

$$C4 = 20.788 * \ln(Glu_{uncor}) - 72.493 \qquad \text{(Eq. 4)}$$

The four parameters above, -0.4962, 1.7269, 20.788 and -72.493 were derived empirically based on the multiple lots of glucose reagents in conjunction with blood samples at different hematocrit levels. FIG. 6 is a plot of the blood glucose concentration vs. the constant C3. FIG. 7 is a plot of the blood glucose concentration vs. the constant C4. The curves were fitted with a logarithmic function. Thus, the "apparent" glucose assay results ($Glu_{uncor}$) can be used to calculate the two constants C3 and C4 using Equations 3 and 4 above. The sample hematocrit level, obtained from Equation 1 from the hemoglobin assay, in combination with C3 and C4 can then be used to calculate the hematocrit correction factor (%Correction) based on Equation 2.

According to one embodiment of the present invention, the output of Equations 1 and 2 above may be used to correct the blood glucose assay bias of the measured glucose concentration, $Glu_{uncor}$, using Equations 5 and 6 below. According to Equations 5 and 6, the correction of the blood glucose assay bias is dependent on whether the percent hematocrit, %Hct, is inside or outside of the normal hemotacrit range of about 35% to about 45%. Specifically, if the %Hct is outside of the normal hematocrit range, the corrected glucose concentration is calculated using:

$$Glu_{cor} = Glu_{uncor} - (Glu_{uncor} \times \%Correction) \qquad \text{Eq. 5}$$

where %Hct is less than about 35% or greater than about 45%.

If the %Hct is inside of the normal hematocrit range, the corrected glucose concentration is calculated using:

$$Glu_{cor} = Glu_{uncor} \qquad \text{Eq. 6}$$

where %Hct is greater than about 35% and less than about 45%. As discussed above, the constants, values and ranges for Equations 1-6 above are dependent on the reagent system being used in the testing device. The reagent system used to develop the Equations 1-6 is based on an electrochemical reaction using glucose oxidase enzyme and potassium ferricyanide mediator. However, the present invention is not limited to a specific enzyme or mediator, and could include other enzymes or mediators typically used in testing devices.

As discussed above, FIG. 3 illustrates one embodiment of the inventive method described herein in for correcting the blood glucose assay bias in a whole blood sample. Generally, the inventive method involves the acts of (1) measuring the glucose concentration, $Glu_{uncor}$, of the blood sample; (2) measuring the total hemoglobin concentration, Hb, of the blood sample; (3) calculating the percent hematocrit, % Hct, of the blood sample using an empirical relationship; (4) calculating the correction factor, % Correction, of the blood sample using an empirical relationship; and (5) calculating the corrected glucose concentration, $Glu_{cor}$, and displaying the corrected glucose concentration, $Glu_{cor}$, to the user of the testing device or system. The calculating acts may be performed by a processor. Thus, by using the inventive method described herein, a blood glucose concentration of a blood sample can be provided to a patient or physician that has been corrected for the hematocrit bias or effect and, hence, a more accurate assessment of the patient's glycemic stage may be obtained.

By using the methods and devices described herein, hematocrit adjustments may be performed during blood glucose testing. By programming the equations described herein into software that is used with an optical or electrochemical device or system, a corrected blood glucose concentration can be calculated and reported to the user. Alternatively, one or more pieces of data obtained from the equations described herein may be manually calculated and/or entered into the software that is used with the optical or electrochemical device or system to provide a corrected blood glucose concentration. The corrected blood glucose concentration will more accurately reflect the true glucose concentrations and, hence, the true glycemic stage of the patient.

Furthermore, the methods described herein may be employed in a single "combo" testing device or system that determines hemoglobin, hemoglobin $A_{1C}$ and glucose concentrations. The system may include a meter and one or more testing devices, such as a one or more test strips, one or more test cartridges, a combination of a test strip and test cartridge, etc. The combination testing device or system may be based on either an optical or electrochemical reaction system and may provide an easier, more convenient and more reliable method for correcting the blood glucose assay bias. Thus, the methods and devices described herein may obviate the need to measure the percent hematocrit and hemoglobin $A_{1C}$ of the blood sample in a clinical or laboratory setting. Because the methods described herein allow a patient to determine an accurate hemoglobin $A_{1C}$ and glucose value from home without waiting on test results from a laboratory or clinic, the patient can immediately relay his or her true glucose value to a physician or other health care professional.

**ALTERNATIVE EMBODIMENTS**

Alternative Process A

A method for correcting the blood glucose assay bias in a testing device or system that measures glucose and hemoglobin concentrations in a blood sample, the method comprising the acts of:

measuring the total hemoglobin concentration, Hb, of the blood sample as part of a hemoglobin $A_{1C}$ assay;
measuring the glucose concentration, $Glu_{uncor}$, of the blood sample;
calculating the percent hematocrit, %Hct, of the blood sample based on an empirical relationship between the total hemoglobin concentration, Hb, and the percent hematocrit, %Hct;
calculating a correction factor, %Correction, based on an empirical relationship between the percent hematocrit, %Hct and the correction factor, %Correction; and
correcting the glucose concentration, $Glu_{uncor}$, of the blood sample using the %Correction to provide a corrected gluclose concentration, $Glu_{cor}$.

Alternative Process B

The method of alternative process A, wherein the empirical relationship between the correction factor, %Correction, and the percent hematocrit, %Hct, is represented by the formula %Correction = (%Hct x C3) + C4, wherein C3 and C4 are constants which are glucose-concentration dependent.

Alternative Process C

The method of alternative process A, wherein the empirical relationship between the percent hematocrit, %Hct, and the total hemoglobin concentration, Hb, is represented by the formula %Hct = (C1 × Hb) + C2, wherein Hb is in g/dL, and C and C2 are constants.

Alternative Process D

The method of alternative process A, wherein, if the percent hematocrit, %Hct, is within a defined normal range, the corrected glucose concentration, $Glu_{cor}$ is calculated according to the relationship $Glu_{cor} = Glu_{uncor}$.

Alternative Process E

The method of alternative process D, wherein the normal range is from about 35% to about 45%.

Alternative Process F

The method of alternative process A, wherein, if the percent hematocrit, %Hct, is outside of a defined normal range, the corrected glucose concentration, $Glu_{cor}$ is calculated according to the relationship $Glu_{cor} = Glu_{uncor} - (Glu_{uncor}$ x %Correction).

Alternative Process G

The method of alternative process F, wherein the normal range is less than about 35% and greater than about 45%.

Alternative Process H

The method of alternative process A, wherein the glucose concentration is performed using an optical testing method.

Alternative Process I

The method of alternative process A, wherein the glucose concentration is performed using an electrochemical testing method.

Alternative Process J

The method of alternative process A, wherein the testing device or system that measures glucose and hemoglobin concentrations in a blood sample includes a single combination monitoring device.

Alternative Process K

The method of alternative process A, wherein the method further calculates the percentage hemoglobin $A_{1C}$, $\%HbA_{1C}$, as a ratio of the concentration of hemoglobin $A_{1C}$ to the concentration of total hemoglobin according to the relationship $\%HbA_{1C} = (HbA_{1C}/Hb) \times 100$.

Alternative Embodiment L

A testing device or system for measuring the blood glucose concentration and total hemoglobin concentration in a blood sample and correcting the measured glucose concentration for a glucose assay bias effect, the testing device or system comprising:

a reagent system adapted to measure the blood glucose concentration, $Glu_{uncor}$ of the blood sample;
a reagent system adapted to measure the total hemoglobin concentration, Hb, of the blood sample as part of a hemoglobin $A_{1C}$ assay;
a processor adapted to calculate

a percent hematocrit, %Hct, of the blood sample based on an empirical relationship using the total hemoglobin concentration, Hb,
a correction factor, %Correction, based on an empirical relationship using the percent hematocrit, %Hct,
a corrected glucose concentration, $Glu_{cor}$, of the blood sample based on the correction factor, %Correction; and

a display adapted to display the corrected glucose concentration, $Glu_{cor}$, to a user.

Alternative Embodiment M

The testing device of alternative embodiment L, wherein the empirical relationship between the correction factor, %Correction, and the percent hematocrit, %Hct, is represented by the formula %Correction = (%Hct x C3) + C4, wherein C3 and C4 are constants which are glucose-concentration dependent.

Alternative Embodiment N

The testing device of alternative embodiment L, wherein the empirical relationship between the percent hematocrit, %Hct, and the total hemoglobin concentration, Hb, is represented by the formula %Hct = (C1 x Hb) + C2, wherein Hb is in mmole/L and C 1 and C2 are constants.

Alternative Embodiment O

The testing device of alternative embodiment L, wherein, if the percent hematocrit, %Hct, is within a defined normal range, the corrected glucose concentration, $Glu_{cor}$. is calculated according to the relationship $Glu_{cor} = Glu_{uncor}$.

Alternative Embodiment P

The testing device of alternative embodiment O, wherein the normal range is from about 35% to about 45%.

Alternative Embodiment Q

The testing device of alternative embodiment L, wherein, if the percent hematocrit, %Hct, is outside of a defined normal range, the corrected glucose concentration, $Glu_{cor}$ is calculated according to the relationship $Glu_{cor} = Glu_{uncor} - (Glu_{uncor} \times \%Correction)$.

Alternative Embodiment R

The testing device of alternative embodiment Q, wherein the normal range is less than about 35% and greater than about 45%.

Alternative Embodiment S

The testing device of alternative embodiment L, wherein the glucose concentration is performed using an optical testing method.

Alternative Embodiment T

The testing device of alternative embodiment L, wherein the glucose concentration is performed using an electrochemical testing method.

Alternative Embodiment U

A single combination testing device adapted for measuring blood glucose concentration and total hemoglobin concentration in a blood sample, the total hemoglobin concentration being measured as part of a hemoglobin $A_{1C}$ assay.

While the invention is susceptible to various modifications and alternative forms, specific embodiments are shown by way of example in the drawings and described in detail. It should be understood, however, that it is not intended to limit the invention to the particular forms disclosed, but on the contrary, the intention is to cover all modifications, equivalents, and alternatives falling within the spirit and scope of the invention as defined by the appended claims.

## Claims

1. A method for correcting the blood glucose assay bias in a testing device or system that measures glucose and hemoglobin concentrations in a blood sample, the method comprising the acts of:

    measuring the total hemoglobin concentration, Hb, of the blood sample as part of a hemoglobin $A_{1C}$ assay;
    measuring the glucose concentration, $Glu_{uncor}$, of the blood sample;
    calculating the percent hematocrit, %Hct, of the blood sample based on an empirical relationship between the total hemoglobin concentration, Hb, and the percent hematocrit, %Hct;
    calculating a correction factor, %Correction, based on an empirical relationship between the percent hematocrit, %Hct and the correction factor, %Correction; and
    correcting the glucose concentration, $Glu_{uncor}$, of the blood sample using the %Correction to provide a corrected gluclose concentration, $Glu_{cor}$.

2. The method of claim 1, wherein the empirical relationship between the correction factor, %Correction, and the percent hematocrit, %Hct, is represented by the formula %Correction = (%Hct x C3) + C4, wherein C3 and C4 are constants which are glucose-concentration dependent.

3. The method of claim 1, wherein the empirical relationship between the percent hematocrit, %Hct, and the total hemoglobin concentration, Hb, is represented by the formula %Hct = (C1 x Hb) + C2, wherein Hb is in g/dL, and C1 and C2 are constants.

4. The method of claim 1, wherein, if the percent hematocrit, %Hct, is within a defined normal range, the corrected glucose concentration, $Glu_{cor}$ is calculated according to the relationship $Glu_{cor} = Glu_{uncor}$.

5. The method of claim 4, wherein the normal range is from about 35% to about 45%.

6. The method of claim 1, wherein, if the percent hematocrit, %Hct, is outside of a defined normal range, the corrected glucose concentration, $Glu_{cor}$ i s calculated according to the relationship $Glu_{cor} = Glu_{uncor} - (Glu_{uncor} \times \%Correction)$.

7. The method of claim 6, wherein the normal range is less than about 35% and greater than about 45%.

8. The method of claim 1, wherein the glucose concentration is performed using an optical testing method.

**9.** The method of claim 1, wherein the glucose concentration is performed using an electrochemical testing method.

**10.** The method of claim 1, wherein the testing device or system that measures glucose and hemoglobin concentrations in a blood sample includes a single combination monitoring device.

**11.** The method of claim 1, wherein the method further calculates the percentage hemoglobin $A_{1C}$, $\%HbA_{1C}$, as a ratio of the concentration of hemoglobin $A_{1C}$ to the concentration of total hemoglobin according to the relationship $\%HbA_{1C} = (HbA_{1C}/Hb) \times 100$.

**12.** A testing device or system for measuring the blood glucose concentration and total hemoglobin concentration in a blood sample and correcting the measured glucose concentration for a glucose assay bias effect, the testing device or system comprising:

a reagent system adapted to measure the blood glucose concentration, $Glu_{uncor}$ of the blood sample;
a reagent system adapted to measure the total hemoglobin concentration, Hb, of the blood sample as part of a hemoglobin $A_{1C}$ assay;
a processor adapted to calculate
a percent hematocrit, %Hct, of the blood sample based on an empirical relationship using the total hemoglobin concentration, Hb,
a correction factor, %Correction, based on an empirical relationship using the percent hematocrit, %Hct,
a corrected glucose concentration, $Glu_{cor}$, of the blood sample based on the correction factor, %Correction; and
a display adapted to display the corrected glucose concentration, $Glu_{cor}$, to a user.

**13.** The testing device of claim 12, wherein the empirical relationship between the correction factor, %Correction, and the percent hematocrit, %Hct, is represented by the formula %Correction = (%Hct x C3) + C4, wherein C3 and C4 are constants which are glucose-concentration dependent.

**14.** The testing device of claim 12, wherein the empirical relationship between the percent hematocrit, %Hct, and the total hemoglobin concentration, Hb, is represented by the formula $\%Hct = (C1 \times Hb) + C2$, wherein Hb is in mmole/L and C1 and C2 are constants.

**15.** The testing device of claim 12, wherein, if the percent hematocrit, %Hct, is within a defined normal range, the corrected glucose concentration, $Glu_{cor}$. is calculated according to the relationship $Glu_{cor} = Glu_{uncor}$.

**16.** The testing device of claim 15, wherein the normal range is from about 35% to about 45%.

**17.** The testing device of claim 12, wherein, if the percent hematocrit, %Hct, is outside of a defined normal range, the corrected glucose concentration, $Glu_{cor}$ is calculated according to the relationship $Glu_{cor} = Glu_{uncor} - (Glu_{uncor} \times \%Correction)$.

**18.** The testing device of claim 17, wherein the normal range is less than about 35% and greater than about 45%.

**19.** The testing device of claim 12, wherein the glucose concentration is performed using an optical testing method.

**20.** The testing device of claim 12, wherein the glucose concentration is performed using an electrochemical testing method.

**21.** A single combination testing device adapted for measuring blood glucose concentration and total hemoglobin concentration in a blood sample, the total hemoglobin concentration being measured as part of a hemoglobin $A_{1C}$ assay.

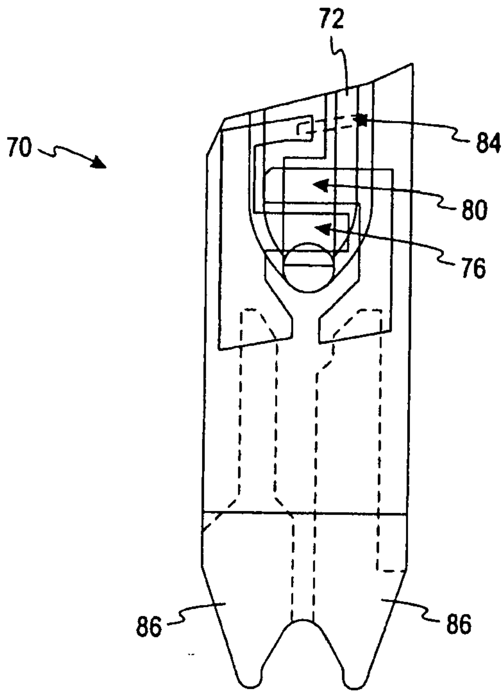


Fig. 1

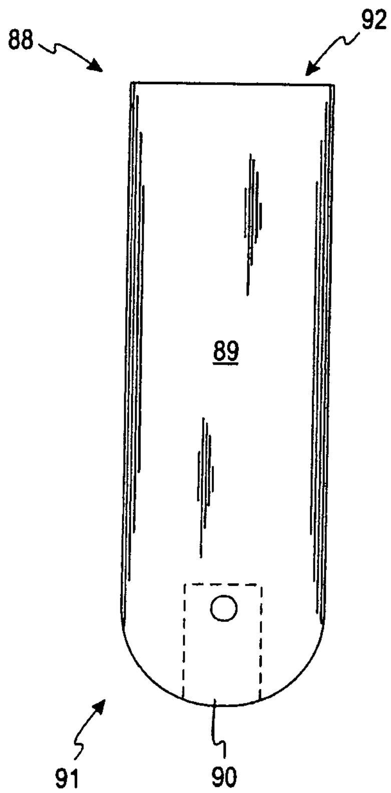


Fig. 2

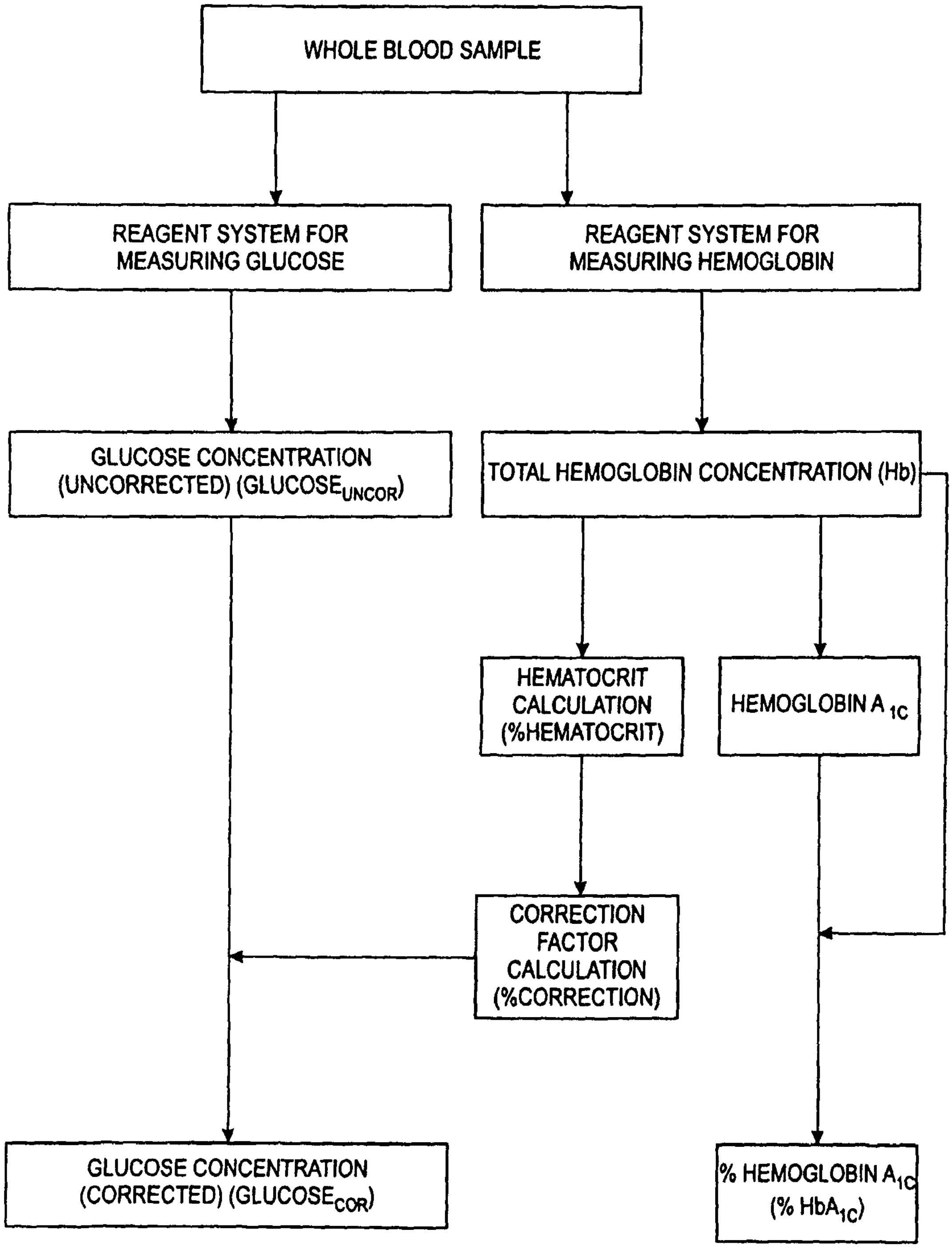
WHOLE BLOOD SAMPLE
REAGENT SYSTEM FOR MEASURING GLUCOSE
REAGENT SYSTEM FOR MEASURING HEMOGLOBIN
GLUCOSE CONCENTRATION (UNCORRECTED) ($GLUCOSE_{UNCOR}$)
TOTAL HEMOGLOBIN CONCENTRATION (Hb)
HEMATOCRIT CALCULATION (%HEMATOCRIT)
HEMOGLOBIN $A_{1C}$
CORRECTION FACTOR CALCULATION (%CORRECTION)
GLUCOSE CONCENTRATION (CORRECTED) ($GLUCOSE_{COR}$)
% HEMOGLOBIN $A_{1C}$ (% $HbA_{1C}$)

*Fig. 3*

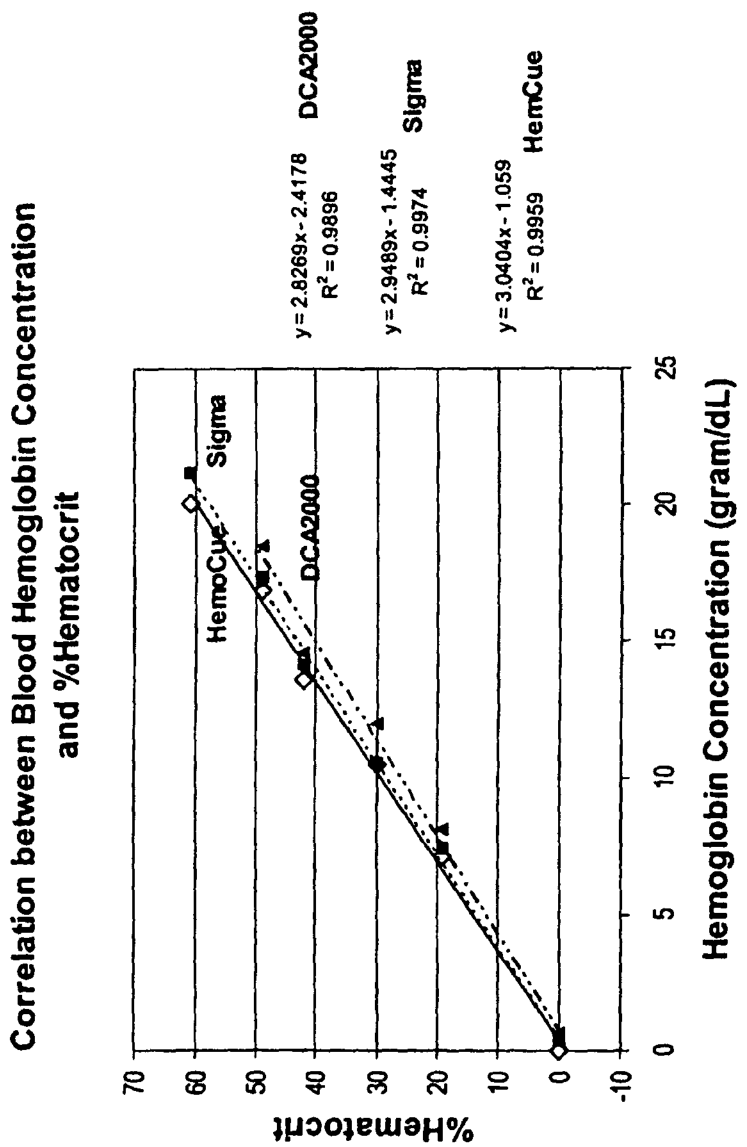
Correlation between Blood Hemoglobin Concentration and %Hematocrit
%Hematocrit
Hemoglobin Concentration (gram/dL)
70
60
50
40
30
20
10
0
-10
0
5
10
15
20
25
HemoCue
Sigma
DCA2000
y = 2.8269x - 2.4178
R² = 0.9896
DCA2000
y = 2.9489x - 1.4445
R² = 0.9974
Sigma
y = 3.0404x - 1.059
R² = 0.9959
HemCue

*Fig. 4*

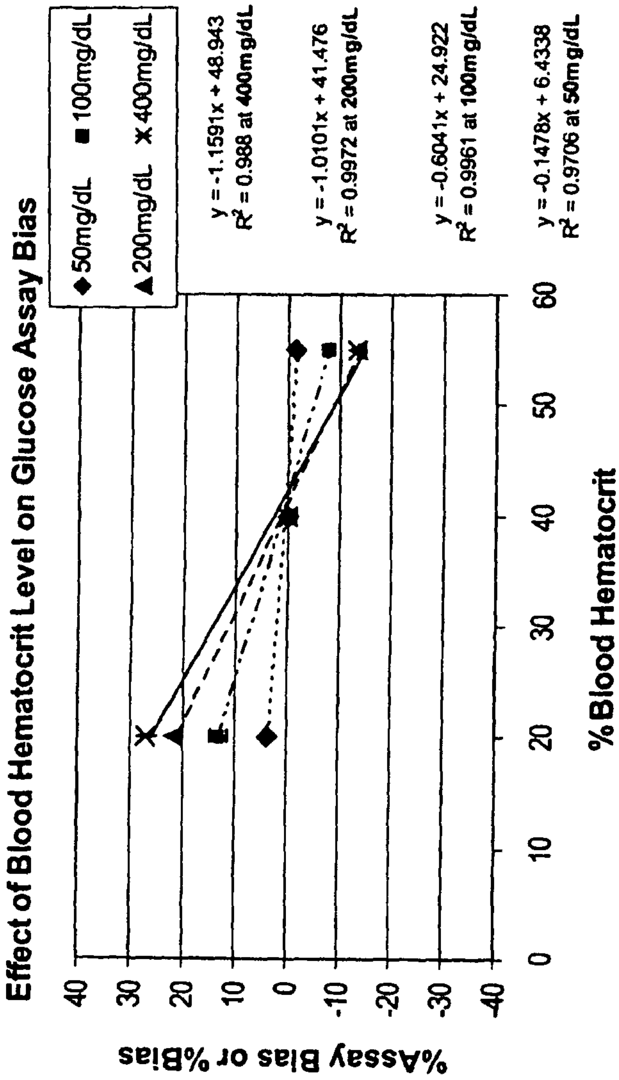
Effect of Blood Hematocrit Level on Glucose Assay Bias
50mg/dL
100mg/dL
200mg/dL
400mg/dL
y = -1.1591x + 48.943
R² = 0.988 at 400mg/dL
y = -1.0101x + 41.476
R² = 0.9972 at 200mg/dL
y = -0.6041x + 24.922
R² = 0.9961 at 100mg/dL
y = -0.1478x + 6.4338
R² = 0.9706 at 50mg/dL
%Assay Bias or %Bias
40
30
20
10
0
-10
-20
-30
-40
0
10
20
30
40
50
60
% Blood Hematocrit

*Fig. 5*

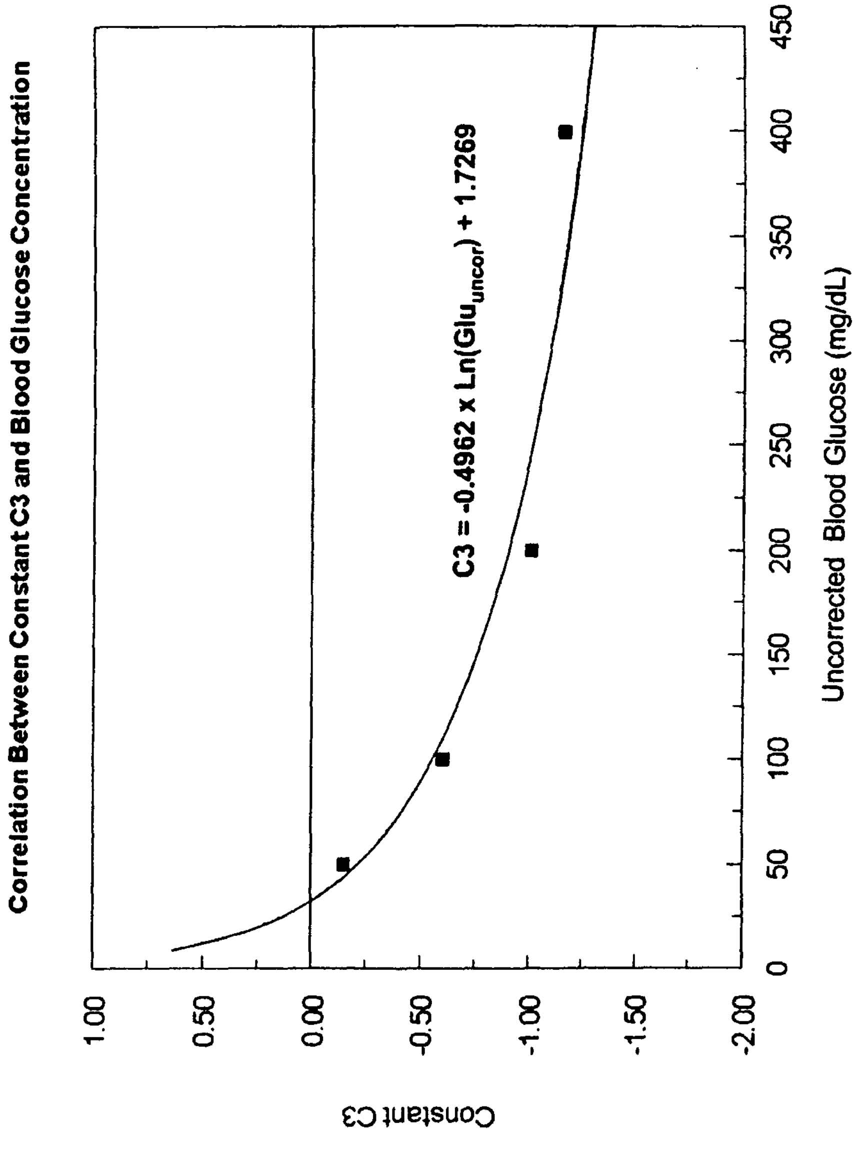
Correlation Between Constant C3 and Blood Glucose Concentration
C3 = -0.4962 x Ln($Glu_{uncor}$) + 1.7269
Constant C3
1.00
0.50
0.00
-0.50
-1.00
-1.50
-2.00
0
50
100
150
200
250
300
350
400
450
Uncorrected Blood Glucose (mg/dL)

*Fig. 6*

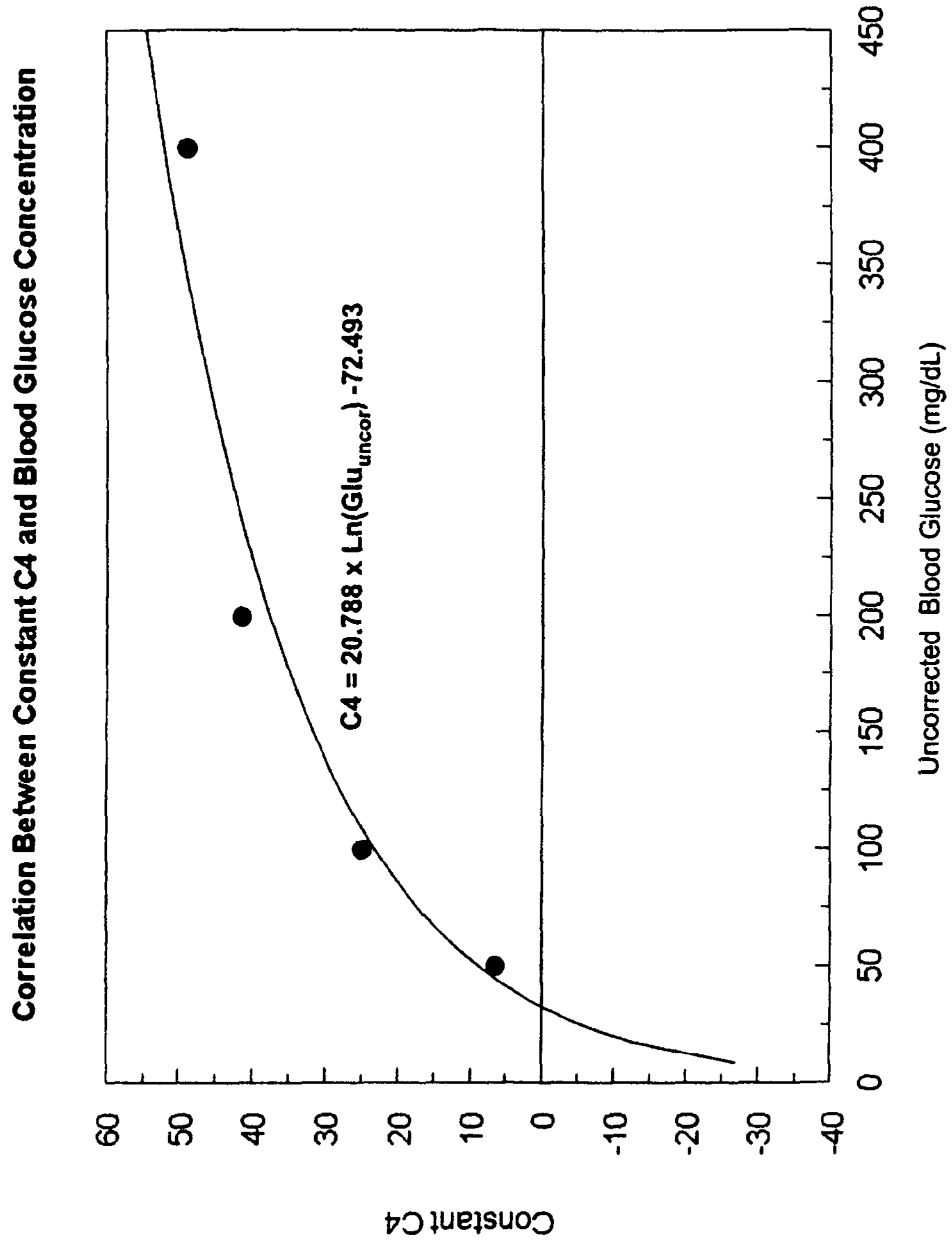

Correlation Between Constant C4 and Blood Glucose Concentration
C4 = 20.788 x Ln(Glu_uncor) -72.493
Constant C4
60
50
40
30
20
10
0
-10
-20
-30
-40
Uncorrected Blood Glucose (mg/dL)
0
50
100
150
200
250
300
350
400
450


*Fig. 7*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6531040 B
- US 5866349 A
- US 5518689 A
- US 5611999 A
- US 5194393 A